Europäisches Patentamt

European Patent Office

Office européen des brevets

(11) Numéro de publication: **0 077 732**
**A1**

(19)

(12) **DEMANDE DE BREVET EUROPEEN**

(21) Numéro de dépôt: **82401903.8**

(22) Date de dépôt: **15.10.82**

(51) Int. Cl.³: **G 01 G 19/04**
**G 01 G 17/06**

(30) Priorité: **19.10.81 FR 8119622**

(43) Date de publication de la demande:
**27.04.83 Bulletin 83/17**

(84) Etats contractants désignés:
**AT BE CH DE GB IT LI LU NL SE**

(71) Demandeur: **M. C. B.**
**11, rue Pierre-Lhomme**
**F-92404 Courbevoie(FR)**

(72) Inventeur: **Christ, Charles**
**29 Avenue Paul Vaillant Couturier**
**F-78390 Bois d'Arcy(FR)**

(72) Inventeur: **Le Gal, Patrick**
**24, rue de la Marne Bâtiment K - Appt. 438**
**F-78800 Houilles(FR)**

(72) Inventeur: **Perrier, Paul**
**28, rue du Parc de Clagny**
**F-78000 Versailles(FR)**

(74) Mandataire: **Lecca, Jean et al,**
**CABINET PLASSERAUD 84, rue d'Amsterdam**
**F-75009 Paris(FR)**

(54) **Procédé et dispositif de pesage programmable d'un mélange ou d'une suspension dont un des constituants est ajouté progressivement, avec agitation périodique des constituants, notamment d'un mélange de sang prélevé progressivement sur un donneur et d'un anticoagulant.**

(57) Le dispositif comprend un plateau (2) apte à recevoir un récipient (3) dans lequel est introduite une certaine quantité d'un premier constituant du mélange ou de la suspension à former, des moyens (16, 17, 18) pour agiter pendant une série de périodes ledit plateau avec le récipient, des moyens (4, 5) pour introduire dans ledit récipient un flux du second constituant du mélange ou de la suspension, des moyens (12, 13, 14, 15, 27) pour peser le plateau avec le récipient et son contenu après chaque période d'agitation, l'introduction de flux du second constituant étant interrompue pendant chaque pesée, et des moyens (22) pour arrêter les opérations alternées d'agitation-introduction et de pesée lorqu'une opération de pesée indique qu'un certain poids prédéterminé, correspondant à l'introduction d'une quantité prédéterminée du second constituant dans le récipient, a été atteint.

Fig.2.

EP 0 077 732 A1

"Procédé et dispositif de pesage programmable d'un mélange ou d'une suspension dont un des constituants est ajouté progressivement, avec agitation périodique des constituants, notamment d'un mélange de sang prélevé progressivement sur un donneur et d'un anticoagulant"

La présente invention concerne un procédé et un dispositif de pesage d'un mélange ou d'une suspension dont un des constituants est ajouté progressivement, avec agitation périodique des constituants.

Elle concerne plus particulièrement un procédé et un dispositif de pesage d'un mélange de sang prélevé progressivement sur un donneur et d'un anticoagulant.

Jusqu'à présent, lorsqu'on voulait réaliser le pesage d'un mélange ou d'une suspension avec agitation, on effectuait séparément le mélange par agitation et le pesage du mélange.

Dans le cas d'une transfusion sanguine, le personnel infirmier devait surveiller l'arrivée du sang dans une bouteille contenant l'anticoagulant pour obtenir empiriquement la proportion désirée $\frac{sang}{anticoagulant}$ et agiter manuellement la bouteille pour réaliser le mélange.

L'invention permet de réaliser automatiquement le mélange, dans la proportion désirée, d'au moins un constituant ajouté de manière progressive (par exemple le sang d'un donneur) et d'au moins un constituant (par exemple l'anticoagulant) préalablement introduit dans le récipient dans lequel s'effectue le mélange, ainsi que la pesée du récipient avec son contenu.

Le procédé selon l'invention consiste - après avoir introduit dans un récipient une certaine quantité d'au moins un constituant, appelé ci-après premier constituant, du mélange ou de la suspension à former - à introduire une quantité prédéterminée d'au moins un autre constituant, appelé ci-après second constituant, ladite quantité prédéterminée étant fonction de la quantité du premier constituant introduite dans le récipient afin de donner une valeur désirée au rapport

$$r = \frac{\text{poids du second constituant}}{\text{poids du premier constituant}},$$

caractérisé en ce qu'on introduit ladite quantité prédéterminée en une série de périodes d'introduction du second constituant et d'agitation du récipient et de son contenu, chacune suivie d'une opération de pesée du récipient et de son contenu, en l'absence d'introduction et d'agitation, jusqu'à ce que l'opération de pesée indique que ladite quantité prédéterminée a été effectivement introduite dans le récipient et donc que le rapport $r$ précité a bien atteint la valeur désirée.

Le procédé s'applique en particulier au cas où le premier constituant est un anticoagulant, par exemple préalablement introduit dans une poche plastique qui constitue le récipient, tandis que le second constituant est le sang d'un donneur.

Le dispositif pour la mise en oeuvre du procédé précité est caractérisé en ce qu'il comprend, en combinaison, un plateau apte à recevoir un récipient dans lequel est introduite une certaine quantité d'un premier constituant du mélange ou de la suspension à former, des moyens pour agiter pendant une série de périodes ledit plateau avec le récipient, des moyens pour introduire dans ledit récipient un flux du second constituant du mélange ou de la suspension, des moyens pour peser le plateau avec le récipient et son contenu après chaque période d'agitation, l'introduction de flux du second constituant étant interrompue pendant chaque pesée, et des moyens pour arrêter les opérations alternées d'agitation-introduction et de pesée lorsqu'une opération de pesée indique qu'un certain poids prédéterminé, correspondant à l'introduction d'une quantité prédéterminée du second constituant dans le récipient, a été atteint.

Dans un mode de réalisation préféré, le dispositif est constitué par un axe fixe, par un plateau recevant un récipient pour le mélange et apte à osciller autour dudit axe fixe, par des moyens pour faire osciller ledit plateau autour dudit axe, ledit plateau venant pendant une portion de chacune de ces oscillations occuper une position déterminée, par des moyens de pesée du plateau et du récipient qu'il supporte, avec son contenu, lorsque le plateau occupe cette position déterminée, et par des moyens pour introduire, seulement lorsque ledit plateau n'occupe pas ladite position déterminée, un flux de liquide dans ledit récipient.

Avantageusement :

- le plateau est solidaire d'au moins une pièce présentant un trou oblong dans lequel s'engage ledit axe fixe et lesdits moyens pour faire osciller ledit plateau sont constitués par une came, telle qu'un excentrique, sur laquelle repose le plateau au moins pendant certaines périodes de temps, et par des moyens pour entraîner en rotation la came;

- les moyens de pesée sont constitués par trois lamelles à jauge de contrainte encastrées en position fixe à l'une de leurs deux extrémités et portant, sur la face supérieure de l'autre de leurs extrémités, un pointeau, le plateau venant reposer sur le pointeau de chacune des trois lames lorsqu'il occupe ladite position déterminée;

- lesdits moyens pour introduire le flux de liquide sont constitués par un tube souple aboutissant dans le récipient et par des moyens pour pincer ledit tube lorsque le plateau occupe ladite position déterminée.

En particulier, dans le cas où les moyens assurant l'oscillation du plateau sont constitués par une came, celle-ci peut agir sur un levier dont une portion pince ledit tube lorsque le plateau occupe ladite position déterminée.

Le dispositif peut en outre comprendre :

- des moyens d'alarme sonore et/ou visuelle d'avertissement de fin d'opération,

- des moyens pour commander le déchargement du récipient,

qui entrent en action lorsque la quantité prédéterminée du second constituant a été introduite dans le récipient et que le dispositif s'arrête d'effectuer les opérations successives d'introduction-agitation et de mélange.

L'invention pourra être bien comprise à l'aide du complément de description qui suit, ainsi que des dessins ci-annexés, lesquels complément et dessins sont, bien entendu, donnés surtout à titre d'indication.

La figure 1 est une vue en perspective, par-dessus, d'un dispositif mettant en oeuvre les perfectionnements selon l'invention.

La figure 2 est une vue schématique, en perspective, du plateau, avec les moyens d'agitation, de pesée et d'introduction du second constituant.

Les figures 3 et 4 sont des vues en élévation latérale, partie latérale du boîtier enlevée, du dispositif montrant le plateau respectivement en position abaissée (de pesage) et soulevée (d'agitation et d'introduction du second constituant).

La figure 5, enfin, est une représentation par blocs de l'ensemble électronique de pesée et de commande des moyens d'agitation du plateau, d'introduction du second constituant et d'alarme.

Selon l'invention et plus spécialement selon celui de ses modes d'application, ainsi que selon ceux des modes de réalisation de ses diverses parties, auxquels il semble qu'il y ait lieu d'accorder la préférence, se proposant, par exemple, de réaliser un procédé et un dispositif de pesage d'un mélange ou d'une suspension dont un des constituants est ajouté progressivement, avec agitation périodique des constituants, notamment d'un mélange de sang prélevé progressivement sur un donneur et d'un anticoagulant, on s'y prend comme suit ou d'une manière analogue.

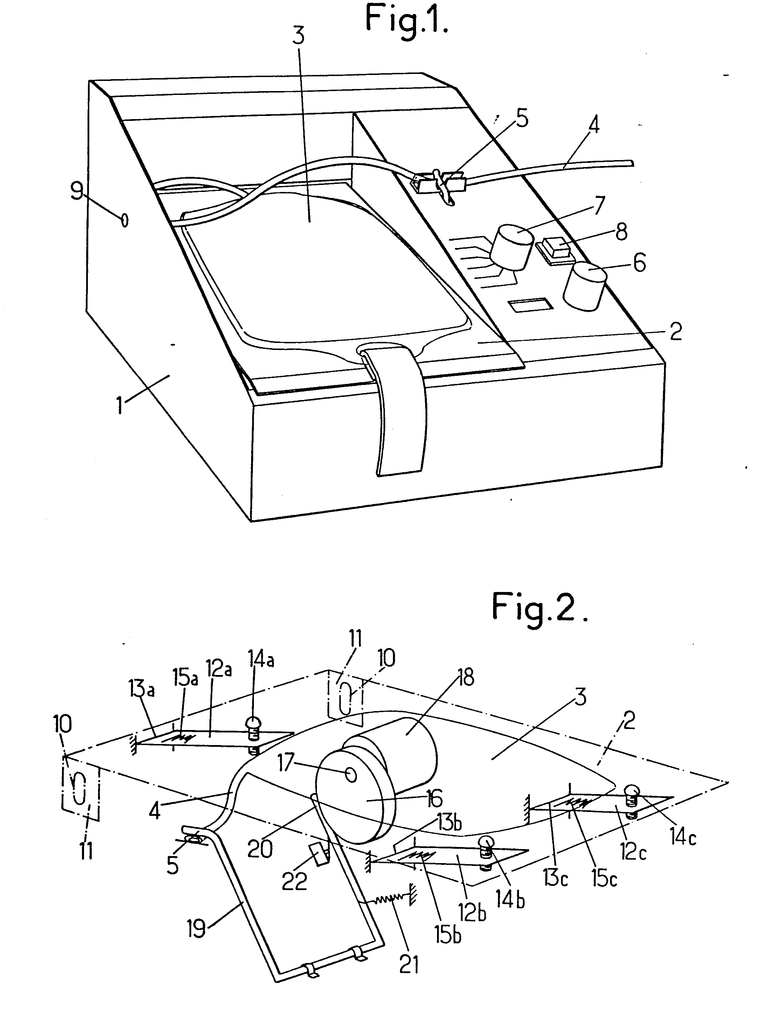

On se réfère tout d'abord aux figures 1 à 4 illustrant le dispositif sous différents aspects.

Sur la figure 1 on voit l'aspect extérieur du dispositif qui comprend un boîtier 1, un plateau 2 qui porte une poche 3 dans laquelle est effectué le mélange ou la suspension désiré, un tube souple 4 permettant l'amenée d'un liquide dans la poche 3, une extrémité 5 d'un étrier ou levier permettant, comme expliqué ci-après, d'interrompre l'arrivée de flux de liquide dans la poche 3 pendant des périodes déterminées, et une série de boutons de commande, notamment un bouton rotatif 6 afin de réaliser le tarage avant la pesée, un bouton rotatif 7 permettant de choisir le poids final à atteindre et un bouton enfonçable bistable, arrêt/marche, 8 permettant d'interrompre volontairement le fonctionnement du dispositif en cas d'incident; dans sa position enfoncée il commande la mise en route du cycle et dans sa position relevée l'arrêt d'urgence ou l'arrêt de l'alarme sonore ou visuelle en fin de cycle.

Considérant maintenant plus particulièrement les figures 2 à 4, on voit que le plateau 2 (illustré en traits interrompus sur la figure 2 pour permettre d'apercevoir les éléments du dispositif qui se trouvent sous le plateau), qui supporte la poche 3 (illustrée en traits fantômes pour les mêmes raisons), est monté oscillant autour d'un axe 9. Cet axe passe à travers les trous oblongs 10 ménagés dans des pièces 11 solidaires du plateau 2. Trois lamelles 12a, 12b, 12c sont encastrées à une de leurs extrémités 13a, 13b, 13c, suivant une ligne fixe et portent au voisinage de leur autre extrémité un pointeau 14a, 14b, 14c. Chaque lamelle 12a, 12b, 12c porte une jauge de contrainte 15a, 15b, 15c qui permet de déterminer le poids du plateau 2 et de ce qui repose sur lui, lorsque ce plateau 2 appuie sur les pointeaux 14a, 14b, 14c en occupant une position déterminée. On n'a pas illustré sur les dessins les moyens électriques ou électroniques permettant de déterminer le poids à partir de la variation de résistance électrique des jauges de contrainte 15a, 15b, 15c provoquée par la déformation des lamelles 12a, 12b, 12c fonction du poids du plateau 2 et de ce qu'il supporte lorsqu'il repose sur les pointeaux 14a, 14b, 14c, ces moyens étant bien connus de l'homme de l'art.

Le dispositif comporte en outre des moyens pour faire osciller le plateau 2 autour de l'axe 9. Ces moyens peuvent être constitués avantageusement par une came 16 ayant par exemple une forme circulaire mais étant montée pour tourner autour d'un axe excentré 17 de manière à constituer un excentrique. La came est entraînée en rotation autour de l'axe excentré 17 par un moteur électrique 18 tournant en continu pendant le fonctionnement du dispositif.

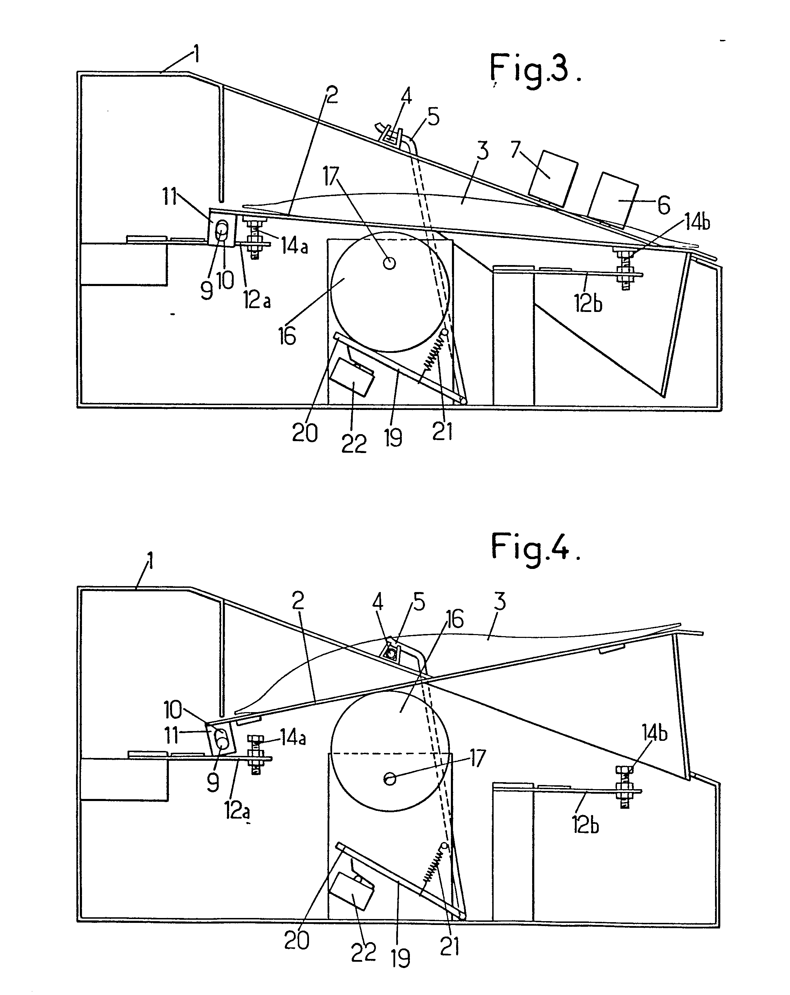

La rotation de la came 16 autour de son axe 17 provoque l'oscillation du plateau 2 entre une position supérieure représentée sur la figure 4 et une position inférieure représentée sur la figure 3. Dans cette position inférieure, appelée également position déterminée, le plateau 2 ne repose plus sur la came 16 mais sur les pointeaux 14a, 14b, 14c et de ce fait les jauges 15a, 15b, 15c permettent de déterminer le poids du plateau 2, de la poche 3 qu'il supporte et du contenu de cette poche dont il sera fait mention ci-après. Par contre pendant la plus grande partie du parcours de la came 2, lorsque le plateau 2 ne repose pas sur les pointeaux 14a, 14b, 14c mais sur la périphérie de la came 16, celui-ci est amené à osciller en agitant le contenu de la poche 3 et en effectuant le mélange du contenu de celle-ci. Dans la position de la figure 4, l'axe 9 est à la partie inférieure des trous oblongs 10, tandis qu'il occupe la partie supérieure de ces trous dans la position de la figure 3 sans que les pièces 11 reposent sur l'axe 9.

Avantageusement, la came 16 agit également sur un levier ou étrier 19 dont une extrémité 20 est sollicitée par un ressort 21 contre la périphérie de la came 16 et dont l'autre extrémité 5 peut pincer le tube 4 afin d'empêcher tout flux liquide de gagner la poche 3 par ce tube.

Le levier ou étrier 19 est construit et disposé de manière que son extrémité 5 obture le tube souple 4 lorsque la came 16 se trouve dans la position représentée sur la figure 3 (position basse), c'est-à-dire lorsque le plateau 2 repose sur les pointeaux 14a, 14b, 14c, donc lorsque le plateau se trouve en position de pesée.

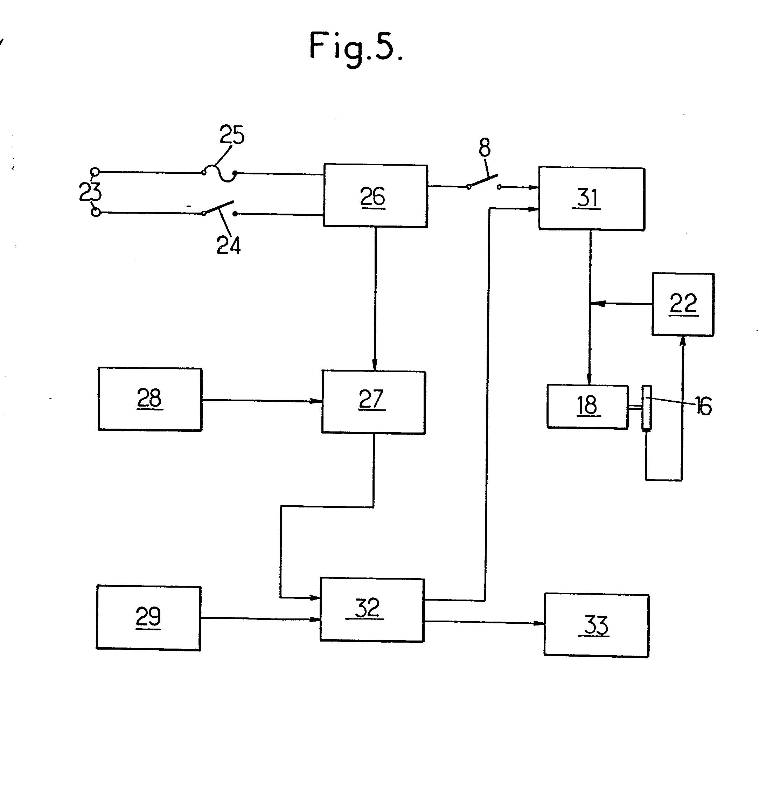

Enfin un interrupteur 22 et un relais faisant partie de l'unité 31 (figure 5) de commande du moteur assurent le maintien,en parallèle,de la tension d'alimentation du moteur 18 et donc sa rotation en continu jusqu'à la fin des opérations successives de pesée.

Le fonctionnement du dispositif qui vient d'être décrit est le suivant, avec référence maintenant également à la figure 5 sur laquelle est illustré l'ensemble du système électrique et électronique de commande, qui est alimenté à partir du secteur 23 au moyen d'un interrupteur général 24, un fusible de protection étant illustré en 25. Le bloc d'alimentation 26, lorsqu'il est alimenté, alimente le pont triple 27 de pesée qui reçoit les informations des trois jauges à contrainte 15a, 15b, 15c.

On introduit tout d'abord dans la poche 3 une quantité déterminée du premier composant, par exemple d'anticoagulant. On réalise alors le tarage du dispositif, c'est-à-dire la pesée du plateau 2, de la poche 3 et de la quantité déterminée du premier composant (tel que l'anticoagulant) qu'elle renferme. Le tarage est effectué par l'unité de tarage 28 commandée par le bouton de tarage 6 que l'on tourne jusqu'à réglage du pont 27.

On affiche ensuite, grâce au bouton 7, dans l'unité électronique 29 de présélection, le poids désiré du deuxième composant (par exemple le sang du donneur). On met alors en marche le moteur 18 en appuyant sur l'interrupteur 8 et en alimentant donc ce moteur 18 par l'unité 31 de commande du moteur. La mise en marche du moteur entraîne la rotation de la came 16 et cette rotation a pour effet de faire osciller le plateau 2.

Il en résulte une série de périodes A pendant lesquelles l'oscillation du plateau 2 agite le contenu de la poche 3 et réalise le mélange du contenu de cette poche constituée par le premier constituant (anticoagulant) introduit initialement et le second constituant (sang du donneur) arrivant par le tube 4 lorsque celui-ci n'est pas pincé par l'extrémité 5 de l'étrier 19. Lorsque la came 16 arrive en position basse, le plateau vient reposer, après chaque période A, sur les pointeaux 14a, 14b, 14c, ce qui réalise une série de périodes B, chaque période B succédant à une période A.

Au cours de chaque période B de pesée, la came 16 agit également par l'étrier 19 sur le tube 4 qui est pincé par l'extrémité 5 de l'étrier 19, ce qui empêche l'arrivée du second constituant dans la poche 3 pendant la pesée.

Celle-ci est effectuée par les jauges de contrainte 15a, 15b, 15c. Un comparateur 32 réalise au moment de chaque pesée la comparaison entre le poids du plateau 2, de la poche 3 et de son contenu, déterminé par le pont triple 27, et le poids à atteindre affiché par le bouton 7 dans l'unité de présélection 29. Lorsque le poids mesuré atteint le poids sélectionné, le comparateur 32 provoque, d'une part, l'ouverture du relais d'alimentation du moteur 18 en agissant sur l'unité 31 de commande du moteur et, d'autre part, une alarme sonore et/ou visuelle par une unité 33 (le comparateur 32 peut également commander automatiquement le déchargement de la poche 3 après l'arrêt du moteur).

Ensuite le dispositif de sécurité 22 actionné par la came 19 commande l'arrêt du moteur directement lorsque la came arrive en position basse suivant les conditions illustrées sur la figure 3.

On voit donc que le dispositif qui vient d'être décrit permet d'obtenir un mélange ou une suspension comportant au moins deux constituants, dont l'un a été introduit initialement dans la poche 3 et dont l'autre est introduit par incréments dans cette poche 3 au moyen du tube 4; ce mélange renferme les deux constituants dans un rapport

$$r = \frac{\text{poids du second constituant}}{\text{poids du premier constituant}}$$

désiré. Ceci est obtenu en effectuant une pesée après l'addition de chaque incrément du second constituant et en agitant la poche 3 entre deux pesées, mais non pendant une pesée, de manière à homogénéiser le mélange ou la suspension. Lorsque la valeur désirée du rapport $r$ est atteinte, le dispositif s'arrête automatiquement. La précision obtenue dépend, d'une part, de la précision des moyens de pesée et, d'autre part, de la quantité du second constituant qui est introduite entre deux pesées. Le degré d'agitation est déterminé par la vitesse de rotation du moteur 18.

Bien entendu on pourrait prévoir d'autres moyens de pesée que des jauges de contrainte, par exemple un système capacitif ou à self induction.

L'invention s'applique particulièrement, mais non exclusivement, au problème du prélèvement et de la conservation du sang, car il permet de recueillir le sang d'un donneur en une quantité désirée dans une poche contenant l'anticoagulant, avec agitation intermittente de la poche, ce qui accélère l'opération de prélèvement du sang, sans danger pour le donneur qui ne fournit que la quantité de sang fixée à l'avance. En pratique on détermine cette quantité et on en déduit la quantité d'anticoagulant correspondante; on introduit cette quantité d'anticoagulant dans la poche 3 avant de commencer le prélèvement du sang; après tarage du plateau 2, avec la poche 3 et la quantité précitée d'anticoagulant, on affiche le poids final à atteindre en ajoutant au poids de tarage le poids du sang du donneur à prélever et on met en marche le dispositif qui s'arrêtera lorsque la quantité de sang fixée à l'avance a été prélevée.

L'invention s'applique également à des mélanges ou suspensions destinés à l'usage pharmaceutique ou plus généralement chimique.

Comme il va de soi et comme il résulte d'ailleurs déjà ce ce qui précède, l'invention ne se limite nullement à ceux de ses modes d'application et de réalisation qui ont été plus spécialement envisagés; elle en embrasse, au contraire, toutes les variantes.

REVENDICATIONS

1. Procédé de pesage d'un mélange ou d'une suspension consistant - après avoir introduit dans un récipient une certaine quantité d'au moins un constituant, appelé ci-après premier constituant, du mélange ou de la suspension à former - à introduire une quantité prédéterminée d'au moins un autre constituant, appelé ci-après second constituant, ladite quantité prédéterminée étant fonction de la quantité du premier constituant introduite dans le récipient afin de donner une valeur désirée au rapport

$$r = \frac{\text{poids du second constituant}}{\text{poids du premier constituant}},$$

caractérisé en ce qu'on introduit ladite quantité prédéterminée en une série de périodes d'introduction du second constituant et d'agitation du récipient et de son contenu, chacune suivie d'une opération de pesée du récipient et de son contenu, en l'absence d'introduction et d'agitation, jusqu'à ce que l'opération de pesée indique que ladite quantité prédéterminée a été effectivement introduite dans le récipient et donc que le rapport $r$ précité a bien atteint la valeur désirée.

2. Procédé selon la revendication 1, caractérisé en ce que le premier constituant est un anticoagulant, tandis que le second constituant est le sang d'un donneur.

3. Dispositif de pesage d'un mélange ou d'une suspension pour la mise en oeuvre du procédé selon la revendication 1 ou 2, caractérisé en ce qu'il comprend, en combinaison, un plateau (2) apte à recevoir un récipient (3) dans lequel est introduite une certaine quantité d'un premier constituant du mélange ou de la suspension à former, des moyens (16, 17, 18) pour agiter pendant une série de périodes ledit plateau avec le récipient, des moyens (4, 5) pour introduire dans ledit récipient un flux du second constituant du mélange ou de la suspension, des moyens (12, 13, 14, 15, 27) pour peser le plateau avec le récipient et son contenu après chaque période d'agitation, l'introduction de flux du second constituant étant interrompue pendant chaque pesée, et des moyens (32, 33, 22) pour arrêter les opérations alternées d'agitation-introduction et de pesée lorsqu'une opération de pesée indique qu'un certain poids prédéterminé, correspondant à l'introduction d'une quantité prédéterminée du second constituant dans le récipient, a été atteint.

4. Dispositif selon la revendication 3, caractérisé en ce qu'il comporte en combinaison un axe fixe (9), un plateau (2) recevant un récipient (3) pour le mélange et apte à osciller autour dudit axe fixe, des moyens (16, 17, 18) pour faire osciller ledit plateau autour dudit axe, ledit plateau venant pendant une portion de chacune de ces oscillations occuper une position déterminée, des moyens de pesée (12, 13, 14, 15, 27) du plateau et du récipient qu'il supporte, avec son contenu, lorsque le plateau occupe cette position déterminée, et des moyens (4, 5) pour introduire, seulement lorsque ledit plateau n'occupe pas ladite position déterminée, un flux de liquide dans ledit récipient.

5. Dispositif selon la revendication 4, caractérisé en ce que le plateau est solidaire d'au moins une pièce (11) présentant un trou oblong (10) dans lequel s'engage ledit axe fixe (9), et lesdits moyens pour faire osciller ledit plateau sont constitués par une came (16), telle qu'un excentrique, sur laquelle repose le plateau (2) au moins pendant certaines périodes de temps, et par des moyens (18) pour entraîner en rotation la came.

6. Dispositif selon la revendication 4 ou 5, caractérisé en ce que les moyens de pesée sont constitués par trois lamelles (12a, 12b, 12c) à jauge de contrainte (15a, 15b, 15c) encastrées en position fixe à l'une de leurs deux extrémités (13a, 13b, 13c) et portant, sur la face supérieure de l'autre de leurs extrémités, un pointeau (14a, 14b, 14c), le plateau (2) venant reposer sur le pointeau de chacune des trois lames lorsqu'il occupe ladite position déterminée.

7. Dispositif selon l'une quelconque des revendications 4, 5 et 6, caractérisé en ce que lesdits moyens pour introduire le flux de liquide sont constitués par un tube souple (4) aboutissant dans le récipient (3) et par des moyens (5, 19, 20, 16) pour pincer ledit tube lorsque le plateau occupe ladite position déterminée.

8. Dispositif selon l'ensemble des revendications 6 et 7, caractérisé en ce que ladite came (16) agit sur un levier (19) dont une portion (5) pince ledit tube (4) lorsque le plateau occupe ladite position déterminée.

9. Dispositif selon l'une quelconque des revendications 3 à 8, caractérisé en ce qu'il comporte en outre des moyens d'alarme sonore et/ou visuelle d'avertissement de fin d'opération, qui entrent en action lorsque la quantité prédéterminée du second constituant a été introduite dans le récipient et que le dispositif s'arrête d'effectuer les opérations successives d'introduction-agitation et de mélange.

10. Dispositif selon l'une quelconque des revendications 3 à 9, caractérisé en ce qu'il comporte en outre des moyens pour commander le déchargement du récipient (3) qui entrent en action lorsque la quantité prédéterminée du second constituant a été introduite dans le récipient et que le dispositif s'arrête d'effectuer les opérations successives d'introduction-agitation du mélange.

# Fig.1.

# Fig.2.

# Fig.3.

# Fig.4.

# Fig.5.

## Office européen des brevets

## RAPPORT DE RECHERCHE EUROPEENNE

EP 82 40 1903

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| | --- | | G 01 G 19/64 |
| A | US-A-2 663 247 (B.HENSGEN et al.) *Revendication 1* | 1,7 | G 01 G 17/06 |
| | --- | | |
| A | US-A-3 557 789 (E.POITRAS) *Abrégé* | 2 | |
| | ----- | | |

DOMAINES TECHNIQUES RECHERCHES (Int. Cl. ³)

G 01 G
B 01 L
A 61 M

Le présent rapport de recherche a été établi pour toutes les revendications

| Lieu de la recherche | Date d'achèvement de la recherche | Examinateur |
|---|---|---|
| LA HAYE | 28-01-1983 | VITZTHUM N.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03.82